# EUROPEAN PATENT SPECIFICATION

(11) **EP 3 332 725 B1**
(45) Date of publication and mention of the grant of the patent: **22.06.2022**
(21) Application number: 16203234.6
(22) Date of filing: 09.12.2016
(51) Int. Cl.: A61B 18/14, A61B 18/00, A61B 18/12

(54) **CONNECTOR, MEDICAL INSTRUMENT AND METHOD FOR MANUFACTURING A MEDICAL INSTRUMENT**
VERBINDER, MEDIZINISCHES INSTRUMENT UND VERFAHREN ZUR HERSTELLUNG DES MEDIZINISCHEN INSTRUMENTS
CONNECTEUR, INSTRUMENT MÉDICAL ET PROCÉDÉ DE FABRICATION D'UN INSTRUMENT MÉDICAL

(43) Date of publication of application: 13.06.2018
(73) Proprietor: Sutter Medizintechnik GmbH, 79108 Freiburg (DE)
(72) Inventor: Weitkamp, Dirk, 79183 Waldkirch (DE)
(74) Representative: Rummler, Felix

(56) References cited:
- DE-A1- 2 841 492
- DE-A1- 3 012 849
- JP-U- H0 520 714
- US-A- 5 925 046
- US-B1- 6 343 961

## Description

### Technical Field

The present invention relates to a connector, a medical instrument and a method for manufacturing a medical instrument.

### Background

Connectors for medical instruments, in particular bipolar electrodes or forceps, are known. Such connectors are used for the assembly of forceps, wherein the arms of the forceps are paired by putting the arms into a housing element of the connector, which is formed as a hollow body. After placing the arms in the housing element, the end portions of the arms are fixed within the housing element, with the result that the arms are held together by the housing element. Usually a bonding agent is added into the housing element after the arms of the forceps are placed into the housing element. By the fixation of the arms within the housing element, a fulcrum point of the forceps is created.

However, a problem of such known connectors emerges during the assembly, when the arms of the medical instrument are to be fixed in the housing element. During this step the arms need to be stably aligned in relation to the housing element, especially during the curing time of the bonding agent. To achieve this, fabrication machines may be needed, which are able to hold the arms during the curing and to align the arms relative to the housing element.

If the arms are not constantly aligned relative to the housing element during the curing, they may shift from their intended position, which impairs the quality of the produced medical instrument. In particular, an incorrect or inaccurate arrangement of the arms leads to an instrument which is not suitable for use in medical treatments.

US 6 343 961 B1 discloses a connecting plug for a bipolar coagulation instrument having an insulating body for mechanically combining and electrically separating and insulating both of its blades or electrical connecting lugs is provided.

DE 30 12 849 A1 describes a bipolar coagulation forceps and a method for producing such.

The present invention aims to address these problems and to provide a connector for a medical instrument, in particular a bipolar electrode or forceps, that enables an improved, especially more accurate, assembly of the medical instrument.

### Summary

The connector according to the invention is characterized by the features according to claim 1. In particular the connector comprises:
at least one alignment element for receiving an end portion of at least one of the arms of the instrument,
thereby to align the arm or arms relative to the alignment element; and
a housing element, in particular a bushing element, for receiving and holding the alignment element, wherein the alignment element is insertable into the housing element.

Accordingly, the connector of the invention addresses the problem of the alignment of the arm or arms of the medical instrument by providing an alignment element. The end portion/s of the arm or arms are received and held by the alignment element already before the alignment element is inserted into the housing element. In an embodiment the housing element may provide means for guiding the alignment element when the alignment element is inserted into the housing element. In such embodiment, the arm or arms are aligned relative to the housing element, in particular without the requirement of additional stabilization of the arm or arms in its/their position by further technical devices.

The connector may be implemented as a socket comprising the at least one alignment element and the housing element.

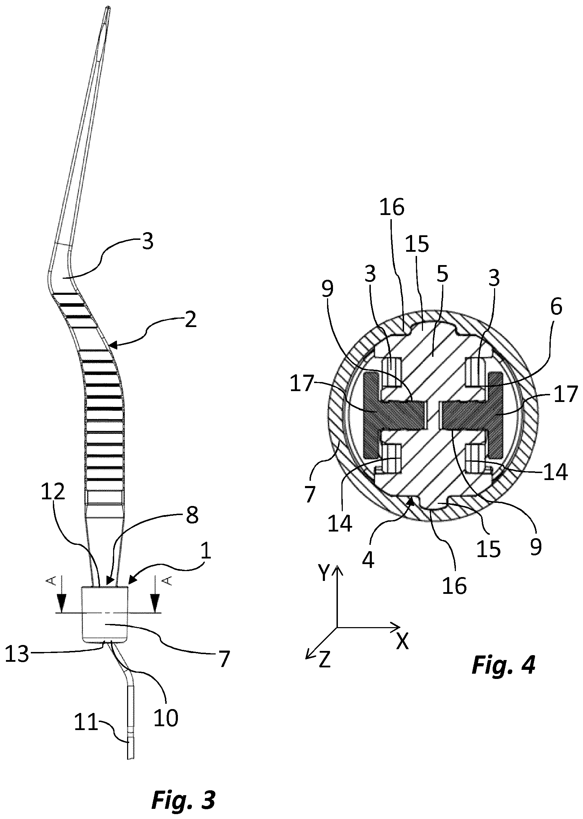

In an embodiment the alignment element of the connector is arranged to align the arm or arms relative to the alignment element in at least two different directions. In particular, the alignment element is arranged to align the arm or arms relative to the alignment element in X- and Y-directions. Preferably, the alignment element is arranged to align the arm or arms relative to the alignment element in X-, Y- and Z-directions (see Fig.4). The alignment element may be held within the housing element by friction. In particular, in a preferred embodiment, the alignment element is insertable into a chamber of the housing element until it reaches a final position, in which position the friction between the alignment element and the housing element is strongest. For example, the alignment element has the shape of a wedge, which is insertable with its smaller end into the chamber of the housing element.

In an embodiment the arm or arms is/are aligned relative to the alignment element before the alignment element is inserted into the housing element. This allows bringing the arm or arms in a stable position relative to the alignment element before inserting the alignment element together with the end portion of the arm or arms into the housing element.

In an embodiment the alignment element has a septum and/or forms a septum within a chamber of the housing element when inserted into the housing element. Preferably of the side edges of the septum are in contact with the inside wall of the housing element, when the alignment element is inserted into the housing element. The septum defines two portions within the chamber, in particular equally sized portions, one for each arm.

The housing element comprises an electrical connection for supplying electric current to the arm or arms of the instrument. Thus, the electric current can be applied for medical treatments.

In another embodiment the alignment element has at least one guiding element, in particular one or more peripheral projections, and the housing element has at least one complementary reception element, in particular one or more recesses located on an inside wall of the housing element, for receiving the guiding element when the alignment element is inserted into the housing element. Preferably, the at least one guiding element and the at least one reception element are formed such that the guiding element is insertable into the receiving element only in one direction. Accordingly, with this configuration it can be prevented that the alignment element is incorrectly inserted into the housing element.

In another embodiment, the alignment element comprises two mounting portions on opposing sides of the alignment element for fixing the end portions of the arms to the alignment element. Especially, each mounting element is for fixing one end portion. Thus, each mounting portion defines a part of the alignment element for receiving and holding an arm. In particular, the mounting portions are shaped such that they define a definite position in which each arm of the medical instrument can be arranged.

According to one embodiment, the connector further comprises a fastening means for fastening the arm or arms to the alignment element. This further stabilizes the alignment of the arm or arms. Preferably the fastening means comprises an aperture or apertures, especially on opposing sides of the alignment element. There may also be a fastening pin or fastening pins insertable into an aperture or apertures in the fastening means and/or the alignment element.

In one embodiment, the housing element and/or the alignment element is/are made from an electrically insulating material.

In one embodiment, the housing element has the shape of a cylinder or a conic section, wherein one of the bases of the cylinder or the conic section forms an opening through which the alignment element is insertable into the housing element, and/or the opposing base is closed except for at least one through hole for one or more electric lines for connecting an external power supply to the arm or arms of the medical instrument. The electric lines comprise one or more electric cables and/or one or more pins to form a plug for connecting with an external electrical power supply.

In another embodiment the alignment element, when inserted in the housing element, is interlocked and/or friction-locked within an inner chamber of the housing element. Thus, the alignment element is fixed within the housing element without the requirement of a bonding agent, like a glue or resin.

In one embodiment the housing element comprises a stop element defining an end position of the alignment element when fully inserted into the housing element. When the alignment element impinges on the stop element the end position is reached.

In another embodiment the alignment element comprises a latch device whereby the alignment element is latched in position when fully inserted in the housing element.

According to another embodiment the closed base of the housing element comprises a sealing element, wherein the at least one through hole is arranged in the sealing element. Preferably, the sealing element is made from a thermoplastic elastomer. The sealing element prevents liquid from entering the housing element through the at least one through hole, since liquid could impair the function of the medical instrument. In addition, the sealing element prevents glue or resin to leak out from the housing element before the glue or resin is fully cured.

Further, the present invention provides a medical instrument, in particular a bipolar electrode or a forceps, comprising a connector as described herein.

Moreover, the present invention also provides a method for manufacturing a medical instrument, in particular a bipolar electrode or a forceps according to claim 12.

The method according to the invention makes it feasible to manufacture a medical instrument solving the problem that the arm or arms have to be aligned correctly before they are fixed within the housing element by using a connector as described above. Hence, the above described advantages pertain also to the method according the invention.

In one embodiment the fixing of the alignment element and the inserted end portion/s of the arm or arms in the end position is performed by adding a bonding agent, preferably a glue and/or a resin, into the chamber of the housing element and curing the bonding agent and/or by using a mechanical fastening system, in particular a mechanical fastening system as described above, e.g. a latch. Preferably, the alignment element is interlocked and/or friction-locked within an inner chamber of the housing element.

The arm or arms of the medical instrument are connected to an electric line, in particular by inserting at least one cable and/or at least one pin through a through hole of the housing element before fixing the alignment element within the housing element.

### Brief Description of the Drawings

The present invention may be better understood from the following description of non-limiting embodiments, with reference to the attached drawings, wherein:
Fig. 1 schematically shows an exploded view of a connector in accordance with an embodiment of the present invention, and components of a medical instrument in accordance with an embodiment of the present invention;
Fig. 2 schematically shows a medical instrument comprising the connector in accordance with an embodiment of the present invention in an assembled state comprising a connector according to an embodiment of the invention;
Figs. 3 schematically shows a top view of the medical instrument of Fig. 2; and
Fig. 4 shows a cross section along line A-A in Fig. 3.

### Detailed Description of Exemplary Embodiments

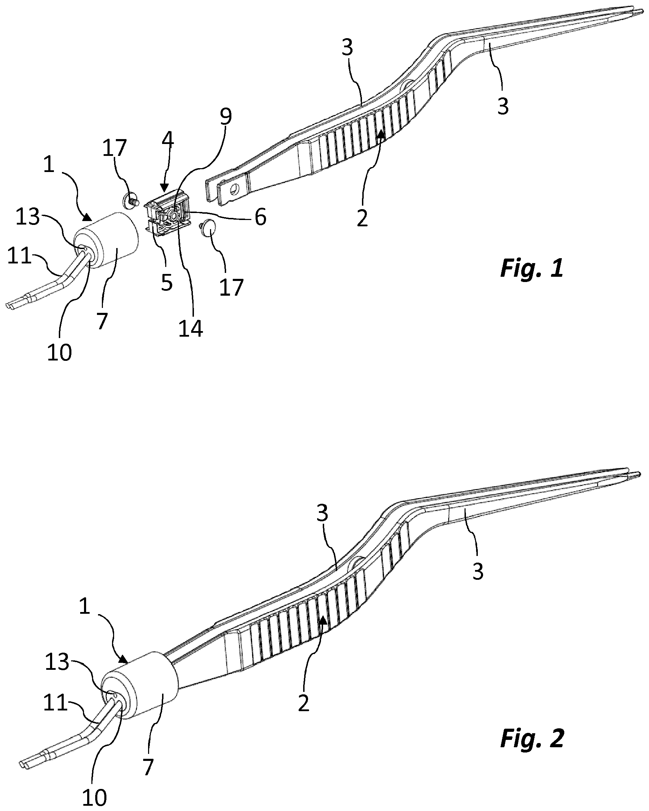

Fig. 1 schematically shows an exploded view of a connector 1 in accordance with an embodiment of the present invention and components of a medical instrument 2 in accordance with an embodiment of the present invention. In particular, the connector 1 is usable for manufacturing a medical instrument 2, such as a bipolar electrode and/or forceps. In the illustrated embodiment, the medical instrument 2 is a forceps that has two arms 3 which are connected with each other in an assembled state, as shown in Figs. 2 and 3. The connector 1 comprises an alignment element 4 and a housing element 7. The housing element 7 can be formed as a bushing element having a round cross-section, as shown in Fig. 1. It may also have a rectangular, hexagonal or any other suitable cross-section. The housing element 7 forms an inner chamber for receiving the alignment element 4.

The alignment element 4 comprises two mounting portions 14 on opposing sides of the alignment element 4 for fixing the end portions of the arms 3 to the alignment element 4. Thereby, the arms 3 are aligned relative to the alignment element 4. In particular, the arms 3 are aligned relative to the alignment element 4 even before the alignment element 4 is inserted into the housing element 7.

The connector 1 further comprises fastening means 6 for fastening end portions of the arms 3 to the alignment element 4. As shown in Figs. 1-4, the fastening means may comprise apertures 9 on opposing sides of the alignment element 4 and two fastening pins 17, which are insertable into the apertures 9 for fixing the end portions of the arms 3 to the alignment element 4, thereby aligning the arms 3 relative to the alignment element 4.

The housing element 7 is arranged for receiving and holding the alignment element 4. The housing element 7 comprises an opening 12, which leads to the inner chamber of the housing element 7. The alignment element 4 is insertable into the housing element 7 through the opening 12, thereby aligning the arms 3 relative to the housing element 7. The housing element 7 is formed as a conic section or a cylinder, wherein one base of the body of the housing element 7 is open and the opposite base is closed. As indicated above, the housing element 7 may also have a rectangular, hexagonal or any other suitable cross-section.

In Fig. 2 and 3 the medical instrument 2 is shown in an assembled configuration. The alignment element 4 is inserted into the housing element 7, whereby the arms 3 are aligned relative to the alignment element 4 and the housing element 7 in X- and Y- directions, preferably in X-, Y- and Z-directions. In the assembled state a fulcrum point 8 of the medical instrument 2 is created by the connector 1.

The alignment element 4 comprises at least one guiding element 15 and the housing element 7 has at least one complementary reception element 16. Thus, the housing element 7 provides a guide for inserting the alignment element 4 into the inner chamber of the housing element 7. As shown in Fig. 4, the guiding element is formed as one or more peripheral projections and the reception element 16 is formed as one or more recesses, which are located on an inside wall of the housing element 7. The reception element 16 is arranged for receiving the guiding element 15 when the alignment element 4 is inserted into the housing element 7. Preferably the at least one guiding element 15 and the at least one reception element 16 are formed such that the guiding element 15 is insertable into the reception element 16 only in one direction.

In the assembled state of the medical instrument 2, the alignment element 4 forms a septum 5 within the inner chamber of the housing element 7, hence, when it is inserted into the housing element 7. Thereby, the septum 5 defines two equally sized parts of the inner chamber, wherein in each part one arm 3 is fixed to the septum 5.

The housing element 7 comprises an electrical connection for supplying electric current to the arms 3 of the instrument 2. One side of the housing element 7 comprises openings. The openings may be placed in a sealing element 13, which prevents liquid from entering the inside of the housing element 7. In addition, the sealing element 13 prevents glue or resin to leak out from the housing element 7 before the glue or resin is fully cured The sealing element 13 comprises a through hole 10 for one or more electric lines 11. The electric lines are arranged for connecting an external power supply to the arms 3 of the medical instrument 2. Preferably, the sealing element 13 is made from a thermoplastic elastomer. As shown in Figs. 1-3, the electric line or lines 11 may comprise electric cables. Alternatively or additionally the electric lines 11 may comprise one or more pins to form a plug for connecting with an external electrical power supply.

The housing element 7 and the alignment element 4 may comprise a latch device. When the alignment element is inserted, in particular fully inserted, into the housing element 7, the alignment element 4 is latched in position.

Therefore, the alignment element 4 and the arms 3 can be fixed within the housing element 7 with or without a bonding agent. Furthermore, a combination of a mechanical fixation with an additional fixation by a bonding agent is also possible.

### Reference signs

1 connector
2 medical instrument
3 arm
4 alignment element
5 septum
6 fastening means
7 housing element
8 fulcrum point
9 aperture
10 through hole
11 electric line
12 opening
13 sealing element
14 mounting portion
15 guiding element
16 reception element
17 fastening pin

## Claims

1. A connector (1) for a medical instrument (2), in particular a bipolar electrode or forceps, the connector (1) comprises at least one alignment element (4) for receiving an end portion of at least one of the arms (3) of the instrument (2), thereby to align the arm (3) or arms (3) relative to the alignment element (4);
wherein the connector (1) further comprises:
- a housing element (7) for receiving and holding the alignment element (4), wherein the alignment element (4) is insertable into the housing element (7), wherein the housing element comprises an opening (12) through which the alignment element (4) is insertable into the housing element (4), and a side of the housing element opposing the opening is closed and comprises at least one through hole (10) for one or more electric lines (11) for connecting an external power supply to the arm (3) or arms (3) of the medical instrument (2), wherein the electric lines (11) comprise one or more electric cables and/or one or more pins to form a plug for connecting with an external electrical power supply,
- wherein the alignment element and the end portion/s of the arm or arms are fixed in an end position in which the alignment element is fully inserted into the housing element.

2. The connector (1) of claim 1, wherein the alignment element (4) is arranged to align the arm (3) or arms (3) relative to the alignment element (4) in at least two different directions, in particular in X- and Y-directions, preferably in X-, Y- and Z-directions, and/or wherein the arm (3) or arms (3) is/are aligned relative to the alignment element (4) when the alignment element (4) is not inserted into the housing element (7) .

3. The connector of claim 1 or 2, wherein the alignment element (4) has a septum (5) and/or forms a septum (5) within an inner chamber of the housing element (7) when inserted into the housing element (7).

4. The connector (1) of any preceding claim, wherein the housing element (7) comprises an electrical connection for supplying electric current to the arm (3) or arms (3) of the instrument (2).

5. The connector (1) according to any one of the preceding claims, wherein the alignment element (4) has at least one guiding element (15), in particular one or more peripheral projections or recesses, and the housing element (7) has at least one complementary reception element (16), in particular one or more recesses or projections, respectively, located on an inside wall of the housing element (7), for receiving the guiding element (15) when the alignment element (4) is inserted into the housing element (7), wherein the at least one guiding element (15) and the at least one reception element (16) are preferably formed such that the guiding element (15) is insertable into the receiving element (16) only in one direction.

6. The connector (1) of any preceding claim, wherein the alignment element (4) comprises two mounting portions (14) on opposing sides of the alignment element (4) for fixing the end portion of the arms (3) to the alignment element (4).

7. The connector (1) according to any one of the preceding claims, further comprising fastening means (6) for fastening the arm (3) or arms (3) to the alignment element (4), wherein the fastening means (6) comprises an aperture (9) or apertures (9) on opposing sides of the alignment element (4), and/or a fastening pin (17) or fastening pins (17) insertable into an aperture (9) or apertures (9) in the alignment element (4) and/or the fastening means (6).

8. The connector (1) according to any one of the preceding claims, wherein the housing element (7) and/or the alignment element (4) is/are made from an electrically insulating material.

9. The connector (1) according to any one of the preceding claims, wherein the alignment element (4), when inserted in the housing element (7), is interlocked and/or friction-locked within an inner chamber of the housing element (7), and/or wherein the housing element (7) comprises a stop element defining an end position of the alignment element (4) when fully inserted into the housing element (7), and/or wherein the housing element (7) and/or the alignment element (4) comprise/s a latch device whereby the alignment element (4) is latched in position when fully inserted in the housing element (7).

10. The connector (1) according to any one of the preceding claims, wherein a or the closed base of the housing element (7), in particular in the shape of a cylinder or conic section, comprises a sealing element (13), preferably made from a thermoplastic elastomer, wherein a or the at least one through hole (10) is arranged in the sealing element (13).

11. A medical instrument (2), in particular a bipolar electrode or a forceps, comprising a connector (1) according to any one of the preceding claim 1 to 10.

12. Method for manufacturing a medical instrument (2), in particular a bipolar electrode or a forceps, comprising the steps:
- providing a connector (1) according to any one of the preceding claim 1 to 10;
- fixing an end portion of an arm (3) or arms (3) of the medical instrument (2) to an alignment element (4), thereby aligning the arm (3) or arms (3) relative to the alignment element (4);
- placing the alignment element (4) and the end portion of the arm (3) or arms (3) into a chamber of a housing element (7) until an end position of the alignment element (4) and the arm (3) or arms (3) is reached within the chamber in which the alignment element is fully inserted into the housing element;
- fixing the alignment element (4) and the inserted end portion of the arm (3) or arms (3) in the end position, wherein the arm (3) or arms (3) of the instrument (2) are connected to an electric line (11), by inserting at least one cable and/or at least one pin through a through hole (10) of the housing element (7) before fixing the alignment element (4) within the housing element (7).

13. Method of claim 12, wherein the fixing of the alignment element (4) and the inserted end portions of the arm (3) or arms (3) in the end position is performed by adding a glue and/or a resin into the chamber of the housing element (7) and curing the glue and/or the resin.

## Patentansprüche

1. Verbinder (1) für ein medizinisches Instrument (2), insbesondere eine bipolare Elektrode oder eine Zange, wobei der Verbinder (1) mindestens ein Ausrichtelement (4) zum Aufnehmen eines Endabschnitts von mindestens einem der Arme (3) des Instruments (2) umfasst, um dadurch den Arm (3) oder die Arme (3) bezogen auf das Ausrichtelement (4) auszurichten; wobei der Verbinder (1) ferner umfasst:
- ein Gehäuseelement (7) zum Aufnehmen und Halten des Ausrichtelements (4), wobei das Ausrichtelement (4) in das Gehäuseelement (7) einsetzbar ist, wobei das Gehäuseelement eine Öffnung (12) umfasst, durch die das Ausrichtelement (4) in das Gehäuseelement (4) einsetzbar ist, und eine Seite des Gehäuseelements gegenüber der Öffnung ist geschlossen und umfasst mindestens ein Durchgangsloch (10) für eine oder mehrere elektrische Leitungen (11) zum Verbinden einer externen Stromversorgung mit dem Arm (3) oder den Armen (3) des medizinischen Instruments (2), wobei die elektrischen Leitungen (11) ein oder mehrere elektrische Kabel und/oder einen oder mehrere Stifte umfassen, um einen Stecker zum Verbinden mit einer externen elektrischen Stromversorgung zu bilden,
- wobei das Ausrichtelement und der/die Endabschnitt/e des Arms oder der Arme in einer Endposition fixiert werden, in der das Ausrichtelement vollständig in das Gehäuseelement eingesetzt ist.

2. Verbinder (1) nach Anspruch 1, wobei das Ausrichtelement (4) dazu angeordnet ist, den Arm (3) oder die Arme (3) bezogen auf das Ausrichtelement (4) in mindestens zwei verschiedenen Richtungen, insbesondere in X- und Y-Richtung, vorzugsweise in X-, Y- und Z-Richtung, auszurichten und/oder wobei der Arm (3) oder die Arme (3) bezogen auf das Ausrichtelement (4) ausgerichtet ist/sind, wenn das Ausrichtelement (4) nicht in das Gehäuseelement (7) eingesetzt ist.

3. Verbinder nach Anspruch 1 oder 2, wobei das Ausrichtelement (4) eine Trennwand (5) hat und/oder eine Trennwand (5) innerhalb einer Innenkammer des Gehäuseelements (7) bildet, wenn es in das Gehäuseelement (7) eingesetzt ist.

4. Verbinder (1) nach einem vorhergehenden Anspruch, wobei das Gehäuseelement (7) eine elektrische Verbindung umfasst, um dem Arm (3) oder den Armen (3) des Instruments (2) elektrischen Strom zuzuführen.

5. Verbinder (1) nach einem der vorhergehenden Ansprüche, wobei das Ausrichtelement (4) mindestens ein Führungselement (15) hat, insbesondere einen oder mehrere periphere Vorsprünge oder Aussparungen, und das Gehäuseelement (7) mindestens ein komplementäres Aufnahmeelement (16) hat, insbesondere eine oder mehrere Aussparungen bzw. Vorsprünge, die sich an einer Innenwand des Gehäuseelements (7) befinden, zum Aufnehmen des Führungselements (15), wenn das Ausrichtelement (4) in das Gehäuseelement (7) eingesetzt wird, wobei das mindestens eine Führungselement (15) und das mindestens eine Aufnahmeelement (16) vorzugsweise so gebildet sind, dass das Führungselement (15) nur in eine Richtung in das Aufnahmeelement (16) einsetzbar ist.

6. Verbinder (1) nach einem vorhergehenden Anspruch, wobei das Ausrichtelement (4) zwei Befestigungsabschnitte (14) auf gegenüberliegenden Seiten des Ausrichtelements (4) zum Fixieren des Endabschnitts des Arms (3) an dem Ausrichtelement (4) umfasst.

7. Verbinder (1) nach einem der vorhergehenden Ansprüche, ferner umfassend ein Befestigungsmittel (6) zum Befestigen des Arms (3) oder der Arme (3) an dem Ausrichtelement (4), wobei das Befestigungsmittel (6) eine Öffnung (9) oder Öffnungen (9) auf gegenüberliegenden Seiten des Ausrichtelements (4) umfasst, und/oder einen Befestigungsstift (17) oder Befestigungsstifte (17), die in eine Öffnung (9) oder Öffnungen (9) in dem Ausrichtelement (4) und/oder dem Befestigungsmittel (6) einsetzbar sind.

8. Verbinder (1) nach einem der vorhergehenden Ansprüche, wobei das Gehäuseelement (7) und/oder das Ausrichtelement (4) aus einem elektrisch isolierenden Material bestehen.

9. Verbinder (1) nach einem der vorhergehenden Ansprüche, wobei das Ausrichtelement (4), wenn in dem Gehäuseelement (7) eingesetzt, innerhalb einer Innenkammer des Gehäuseelements (7) verriegelt und/oder kraftschlüssig verriegelt ist, und/oder wobei das Gehäuseelement (7) ein Anschlagelement umfasst, das eine Endposition des Ausrichtelements (4) definiert, wenn vollständig in das Gehäuseelement (7) eingesetzt, und/oder wobei das Gehäuseelement (7) und/oder das Ausrichtelement (4) eine Rastvorrichtung umfassen, wodurch das Ausrichtelement (4) in Position verrastet ist, wenn vollständig in dem Gehäuseelement (7) eingesetzt.

10. Verbinder (1) nach einem der vorhergehenden Ansprüche, wobei eine oder die geschlossene Basis des Gehäuseelements (7), insbesondere in Form eines Zylinders oder konischen Abschnitts, ein Abdichtungselement (13) umfasst, das vorzugsweise aus einem thermoplastischen Elastomer besteht, wobei ein oder das mindestens eine Durchgangsloch (10) in dem Abdichtungselement (13) angeordnet ist.

11. Medizinisches Instrument (2), insbesondere eine bipolare Elektrode oder eine Zange, umfassend einen Verbinder (1) nach einem der vorhergehenden Ansprüche 1 bis 10.

12. Verfahren zur Herstellung eines medizinischen Instruments (2), insbesondere einer bipolaren Elektrode oder einer Zange, umfassend die Schritte:
- Bereitstellen eines Verbinders (1) nach einem der vorhergehenden Ansprüche 1 bis 10;
- Fixieren eines Endabschnitts eines Arms (3) oder der Arme (3) des medizinischen Instruments (2) an einem Ausrichtelement (4) und dadurch Ausrichten des Arms (3) oder der Arme (3) bezogen auf das Ausrichtelement (4);
- Platzieren des Ausrichtelements (4) und des Endabschnitts des Arms (3) oder der Arme (3) in einer Kammer eines Gehäuseelements (7), bis eine Endposition des Ausrichtelements (4) und des Arms (3) oder der Arme (3) innerhalb der Kammer, in der das Ausrichtelement vollständig in das Gehäuseelement eingesetzt wird, erreicht ist;
- Fixieren des Ausrichtelements (4) und des eingesetzten Endabschnitts des Arms (3) oder der Arme (3) in der Endposition, wobei der Arm (3) oder die Arme (3) des Instruments (2) mit einer elektrischen Leitung (11) verbunden werden, durch Einsetzen mindestens eines Kabels und/oder mindestens eines Stifts durch ein Durchgangsloch (10) des Gehäuseelements (7) vor dem Fixieren des Ausrichtelements (4) in dem Gehäuseelement (7) .

13. Verfahren nach Anspruch 12, wobei das Fixieren des Ausrichtelements (4) und der eingesetzten Endabschnitte des Arms (3) oder der Arme (3) in der Endposition durch Hinzufügen eines Klebemittels und/oder eines Harzes in die Kammer des Gehäuseelements (7) und Aushärten des Klebemittels und/oder des Harzes durchgeführt wird.

## Revendications

1. Connecteur (1) pour un instrument médical (2), en particulier une électrode bipolaire ou pince, le connecteur (1) comprend au moins un élément d'alignement (4) pour recevoir une partie terminale d'au moins un des bras (3) de l'instrument (2), de manière à aligner le bras (3) ou les bras (3) par rapport à l'élément d'alignement (4) ; dans lequel le connecteur (1) comprend en outre :
- un élément de boîtier (7) pour recevoir et maintenir l'élément d'alignement (4), dans lequel l'élément d'alignement (4) est insérable dans l'élément de boîtier (7), dans lequel l'élément de boîtier comprend une ouverture (12) à travers laquelle l'élément d'alignement (4) est insérable dans l'élément de boîtier (4), et un côté de l'élément de boîtier opposé à l'ouverture est fermé et comprend au moins un trou traversant (10) pour une ou plusieurs lignes électriques (11) pour la connexion d'une alimentation en énergie externe au bras (3) ou aux bras (3) de l'instrument médical (2), dans lequel les lignes électriques (11) comprennent un ou plusieurs câbles électriques et/ou une ou plusieurs broches pour former une fiche pour la connexion à une alimentation en énergie électrique externe,
- dans lequel l'élément d'alignement et la ou les parties terminales du bras ou des bras sont fixés dans une position terminale dans laquelle l'élément d'alignement est entièrement inséré dans l'élément de boîtier.

2. Connecteur (1) selon la revendication 1, dans lequel l'élément d'alignement (4) est disposé de manière à aligner le bras (3) ou les bras (3) par rapport à l'élément d'alignement (4) dans au moins deux directions différentes, en particulier dans les directions X et Y, de préférence dans les directions X, Y et Z, et/ou dans lequel le bras (3) ou les bras (3) est/sont alignés par rapport à l'élément d'alignement (4) lorsque l'élément d'alignement (4) n'est pas inséré dans l'élément de boîtier (7).

3. Connecteur selon la revendication 1 ou 2, dans lequel l'élément d'alignement (4) comprend un septum (5) et/ou forme un septum (5) à l'intérieur d'une chambre intérieure de l'élément de boîtier (7) lorsqu'il est inséré dans l'élément de boîtier (7).

4. Connecteur (1) selon une quelconque revendication précédente, dans lequel l'élément de boîtier (7) comprend une connexion électrique pour fournir du courant électrique au bras (3) ou aux bras (3) de l'instrument (2).

5. Connecteur (1) selon l'une quelconque des revendications précédentes, dans lequel l'élément d'alignement (4) comporte au moins un élément de guidage (15), en particulier une ou plusieurs saillies ou cavités périphériques, et l'élément de boîtier (7) comporte au moins un élément de réception complémentaire (16), en particulier une ou plusieurs cavités ou saillies, respectivement, situées sur une paroi intérieure de l'élément de boîtier (7), pour recevoir l'élément de guidage (15) lorsque l'élément d'alignement (4) est inséré dans l'élément de boîtier (7), dans lequel le au moins un élément de guidage (15) et le au moins un élément de réception (16) sont de préférence formés de telle sorte que l'élément de guidage (15) est uniquement insérable dans l'élément récepteur (16) dans une direction.

6. Connecteur (1) selon une quelconque revendication précédente, dans lequel l'élément d'alignement (4) comprend deux parties de montage (14) sur des côtés opposés de l'élément d'alignement (4) pour fixer la partie terminale des bras (3) à l'élément d'alignement (4).

7. Connecteur (1) selon l'une quelconque des revendications précédentes, comprenant en outre des moyens d'amarrage (6) pour amarrer le bras (3) ou les bras (3) à l'élément d'alignement (4), dans lequel les moyens d'amarrage (6) comprennent une ouverture (9) ou des ouvertures (9) sur des côtés opposés de l'élément d'alignement (4), et/ou une broche d'amarrage (17) ou des broches d'amarrage (17) insérables dans une ouverture (9) ou des ouvertures (9) dans l'élément d'alignement (4) et/ou les moyens d'amarrage (6).

8. Connecteur (1) selon l'une quelconque des revendications précédentes, dans lequel l'élément de boîtier (7) et/ou l'élément d'alignement (4) est/sont fabriqués à partir d'un matériau électriquement isolant.

9. Connecteur (1) selon l'une quelconque des revendications précédentes, dans lequel l'élément d'alignement (4), lorsqu'il est inséré dans l'élément de boîtier (7), est imbriqué et/ou verrouillé par friction dans une chambre intérieure de l'élément de boîtier (7), et/ou dans lequel l'élément de boîtier (7) comprend un élément d'arrêt définissant une position terminale de l'élément d'alignement (4) lorsqu'il est entièrement inséré dans l'élément de boîtier (7), et/ou dans lequel l'élément de boîtier (7) et/ou l'élément d'alignement (4) comprend/comprennent un dispositif de blocage par lequel l'élément d'alignement (4) est bloqué en position lorsqu'il est complètement inséré dans l'élément de boîtier (7).

10. Connecteur (1) selon l'une quelconque des revendications précédentes, dans lequel une ou la base fermée de l'élément de boîtier (7), en particulier sous la forme d'une section cylindrique ou conique, comprend un élément d'étanchéité (13), de préférence constitué d'un élastomère thermoplastique, dans lequel un ou le au moins un trou traversant (10) est disposé dans l'élément d'étanchéité (13).

11. Instrument médical (2), en particulier une électrode bipolaire ou une pince, comprenant un connecteur (1) selon l'une quelconque des revendications précédentes 1 à 10.

12. Procédé de fabrication d'un instrument médical (2), en particulier une électrode bipolaire ou une pince, comprenant les étapes consistant à :
- fournir un connecteur (1) selon l'une quelconque des revendications précédentes 1 à 10 ;
- fixer une partie terminale d'un bras (3) ou de bras (3) de l'instrument médical (2) à un élément d'alignement (4), de manière à aligner le bras (3) ou les bras (3) par rapport à l'élément d'alignement (4) ;
- placer l'élément d'alignement (4) et la partie terminale du bras (3) ou des bras (3) dans une chambre d'un élément de boîtier (7) jusqu'à ce qu'une position terminale de l'élément d'alignement (4) et du bras (3) ou des bras (3) soit atteinte à l'intérieur de la chambre dans laquelle l'élément d'alignement est entièrement inséré dans l'élément de boîtier ;
- fixer l'élément d'alignement (4) et la partie terminale insérée du bras (3) ou des bras (3) dans la position terminale, dans lequel le bras (3) ou les bras (3) de l'instrument (2) sont reliés à une ligne électrique (11), en insérant au moins un câble et/ou au moins une broche à travers un trou traversant (10) de l'élément de boîtier (7) avant de fixer l'élément d'alignement (4) à l'intérieur de l'élément de boîtier (7).

13. Procédé selon la revendication 12, dans lequel la fixation de l'élément d'alignement (4) et des parties terminales insérées du bras (3) ou des bras (3) dans la position terminale est effectuée par l'ajout d'une colle et/ou d'une résine dans la chambre de l'élément de boîtier (7) et le durcissement de la colle et/ou de la résine.
